# EUROPEAN PATENT APPLICATION

(11) **EP 2 434 733 A1**
(43) Date of publication of application: **28.03.2012**
(21) Application number: 11007732.8
(22) Date of filing: 22.09.2011
(51) Int. Cl.: H04M 1/60, H04M 1/725, G08G 1/005

(54) **Navigation using a headset having an integrated sensor**

(30) Priority: 27.09.2010 US 890765
(71) Applicant: Sony Ericsson Mobile Communications AB, 221 88 Lund (SE)
(72) Inventor: Agevik, Markus, 21230 Malmö (SE)
(74) Representative: Banzer, Hans-Jörg

(57) **Abstract**

A wireless communication device is configured to communicate signals with a headset worn by a user. The headset includes a sensor that measures the orientation of a user's head to determine which direction the user is looking. The sensor sends that information to the user's device. The device uses the orientation information and a determined geographical position of the user to predict a destination location for the user.

## Description

### FIELD OF THE INVENTION

The present invention is directed generally to wireless communication devices, and particularly to wireless communication devices configured to communicate with a hands-free headset.

### BACKGROUND

Cellular communication devices, such as cellular telephones, are commonly used by many people. Most devices are equipped to perform a variety of different functions that a user would find helpful. One such function, for example, allows a user to determine his or her geographical position. Particularly, many cellular devices include a Global Positioning Satellite (GPS) receiver that receives positioning signals from a plurality of orbiting satellites. Based on these signals, a user's device can determine its geographical position usually to within a few meters. For devices that are not equipped with a GPS receiver, a server in a wireless communication network can triangulate the device's position based on the time-of-arrival of transmitted signals, for example, and provide the positioning information to the device. In some cases, the user's device has the capability of triangulating its own geographical position.

Knowing the user's location is useful for a wide variety of applications. One exemplary application is for navigation. Particularly, some devices are able to provide directions to a predetermined destination location from a user's current position, and then monitor and display the user's progress enroute to the destination. However, in some situations, a user's destination location may not be known *a priori.* For example, consider a situation where a user is walking through an unfamiliar city to sightsee. The user may not always know where certain landmarks are located, or how to navigate to those locations. In such cases, it is difficult for conventional devices to accurately predict where the user is headed, and thus, difficult to assist the user is traveling to a destination. If a device could accurately predict a destination location for a user, it would allow the device to offer directions to the location as well as inform the user as to certain landmarks along the way that the user might find interesting.

### SUMMARY

The present invention provides a wireless communication device and method that selectively sends the audio signals associated with an incoming call to a desired loudspeaker. In one embodiment, the wireless communication device is configured to communicate with a wireless headset worn by a user. The wireless communication device comprises a short-range communication interface configured to receive orientation information from the headset via a short-range communication link and a controller. The controller is programmed or configured to determine a current geographical position of the user, determine which direction the user is looking based on the orientation information, and predict a destination location for the user based on the user's current geographical position and the determined direction information.

In one embodiment, the controller is configured to receive the orientation information via the short-range communication interface from an orientation sensor integrated within the headset.

In one embodiment, the controller is configured to send a request for the orientation information to the headset via the short-range communication interface, and receive the orientation information from the orientation sensor via the short-range communication interface responsive to the request.

In one embodiment, the controller is further configured to generate a first list of locations comprising one or more points of interest based on the user's current geographical position.

In such cases, the controller may further be configured to filter the generated list of locations based on the orientation information to identify a second list of locations comprising one or more points of interest.

The controller may also be configured to select the destination location for the user from the second list of locations based on the user's current geographical position and the orientation information.

In one embodiment, the wireless communication device also includes a display. The controller can be configured to generate and display directions to a selected destination location responsive to user input.

In one embodiment, the controller is further configured to generate directions to a selected destination location and send the directions to a display associated with a head-mounted display.

In one embodiment, the short-range communication interface comprises a wireless interface.

The present invention also provides a method of predicting a destination location for a user. In one embodiment, predicting the destination location comprises establishing a short-range communication link with a wireless headset worn by a user, determining which direction the user is looking based on orientation information received from the headset via the established short-range communication link, and predicting a destination location for the user based on a current geographical position of the user and on the orientation information received from the headset.

In one embodiment, predicting the destination location further comprises generating a request to obtain the orientation information from an orientation sensor integrated within the headset, sending the request to the orientation sensor, and receiving the orientation information from the orientation sensor responsive to the request.

In one embodiment, predicting a destination location for the user comprises generating a first list of locations comprising one or more points of interest based on the user's current geographical position for the user.

In one embodiment, predicting a destination location for the user further comprises filtering the first list of locations based on the orientation information received from the headset to identify a second list of locations for the user.

In one embodiment, predicting a destination location for the user further comprises selecting a destination location from the second list of one or more points of interest based on the user's current geographical position and the orientation information.

In one embodiment, the method further comprises generating and displaying directions to a selected destination location responsive to user input.

The present invention also provides a hands-free headset for a wireless communication device. In one embodiment, the headset comprises a microphone, a speaker, an integrated direction sensor configured to generate orientation information indicating which direction a user is looking based on a detected orientation of the user's head, and a short-range communication interface configured to communicate the orientation information to a wireless communication device.

In one embodiment, the orientation sensor comprises at least one of a compass, a gyroscope, and an accelerometer.

In one embodiment, the hands-free headset further comprises a controller configured to control the short-range communication interface to transmit the orientation information to the wireless communication device responsive to receiving a request for the orientation information from the wireless communication device.

In one embodiment, the short-range communication interface comprises a wireless interface.

In one embodiment, the hands-free headset comprising a head-mounted display that displays text and/or images received from the wireless communication device.

The present invention also provides a method of predicting a destination location for a user of a wireless communication device. In one embodiment, the method comprises establishing a short-range communication link between a user's wireless communication device and a hands-free headset worn by the user, detecting an orientation of the user's head using a sensor in the headset, generating orientation information indicating which direction the user is looking based on the detected orientation of the user's head, and transmitting the orientation information to the wireless communication device via the short-range communication link.

In one embodiment, transmitting the orientation information comprises transmitting the orientation information to the wireless communication device responsive to receiving a request for the orientation information from the wireless communication device.

### BRIEF DESCRIPTION OF THE DRAWINGS

Figure 1 is a perspective view of a wireless communication device operating in a communication system according to one embodiment of the present invention.

Figure 2 is a block diagram illustrating some of the components of a wireless communication device, and a corresponding hands-free headset, according to one embodiment of the present invention.

Figure 3 is a flow diagram illustrating a method of predicting a destination location and one or more points of interest for a user according to one embodiment of the present invention.

Figure 4 is a flow diagram illustrating a method by which a wireless communication device configured according to one embodiment of the present invention predicts the destination location and one or more points of interest for a user.

Figures 5A-5B are perspective views illustrating other embodiments of the present invention.

### DETAILED DESCRIPTION

The present invention provides a wireless communication device that generates a list of possible points of interest for a user. The device then predicts which of these points a user might be interested in and offers directions to that location. The device generates the list and predicts a possible destination location for the user based on the user's current geographical position and information that indicates which direction the user is currently looking. Knowing the user's current location will facilitate the device's ability to obtain information regarding points or locations of possible interest to the user. However, augmenting that knowledge with an understanding of what the user is probably looking at will allow the device to more accurately predict where a user is likely headed. Once this information can be accurately predicted, the device will be able to offer relevant suggestions for directions, as well as information regarding the points that a user may be interested in.

In one embodiment, the user wears a wireless hands-free headset having an orientation sensor, such as a compass, integrated within the headset. The orientation sensor senses the movement of the user's head as the user looks in different directions (i.e., North, South, East, West, etc.), and generates orientation information that indicates the direction the user is looking. That orientation information is then sent to the user's wireless communication device via an established short-range wireless communications link. The user's device, which determines the user's geographical position using GPS, for example, then uses both the geographical position of the user and the orientation information received from the headset to predict one or more nearby points or locations that the user may be interested in visiting.

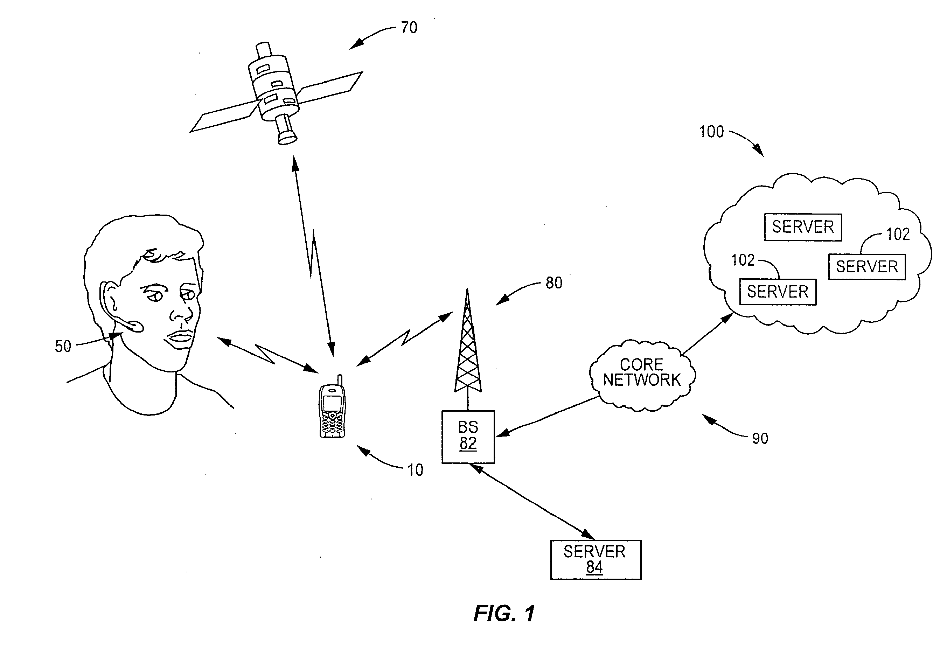

Turning now to Figure 1, a wireless communication device and corresponding peripheral device configured according to one embodiment of the present invention is shown operating in a suitable communication system. As seen in Figure 1 and as described in the corresponding specification, the wireless communication device comprises a cellular telephone 10 and the peripheral device comprises a hands-free headset 50. It should be noted however, that although a cellular telephone 10 and headset 50 are illustrated herein, it is only for illustrative purposes. The present invention is also suitable for use in other communications devices including, but not limited to, satellite telephones, Personal Digital Assistants (PDAs), and computing devices such as laptop and notebook computers. Additionally, the hands-free headset 50 need not comprise a wireless headset that communicates with cellular telephone 10 via a short-range wireless communications link. As seen in more detail later, the present invention is also suitable for operation with headsets that connect to cellular telephone 10 using wires or cables.

Cellular telephone 10 and headset 50 communicate with each other via a well-known short-range wireless protocol, such as the BLUETOOTH protocol, for example. Initially, the cellular telephone 10 and headset 50 execute a procedure to pair with each other and establish a short-range communication link between them. That procedure is well-known to those of ordinary skill in the art and not germane to the present invention. Therefore, it is not discussed in detail herein. It is sufficient to understand that, once paired, headset 50 converts signals received at its microphone into signals compatible with a short-range wireless protocol and transmits the converted signals to the cellular telephone 10. Headset 50 also converts wireless signals received from the cellular telephone 10 to signals compatible with a speaker in headset 50.

In one embodiment of the present invention, which is seen later in more detail, the headset 50 includes an integrated orientation sensor that monitors the orientation of the user's head while the user wears the headset 50. Based on the geographical coordinates of the cellular telephone's 10 current position, and on the orientation information indicating the direction in which the user is looking, cellular telephone 10 can accurately predict what landmark a user is likely to be currently viewing. Given this information, the user's device 10 can then predict where a user might be headed and/or offer suggestions as to possible destination locations for the user.

Cellular telephone 10 may be capable of communicating with a plurality of different types of communication networks. For example, as seen in Figure 1, cellular telephone 10 may be configured to receive navigational signals from a plurality of Global Positioning Satellites (GPS) 70 orbiting above the Earth. Using these signals, cellular telephone 10 can perform some well-known computations to determine its geographical position on the Earth's surface to within a few tens of meters. Cellular telephone 10 may also be configured to communicate voice and/or data with one or more remote parties via a wireless communications network 80 having one or more Base Stations (BS) 82, and with one or more servers 102 in a cloud network 100 via a Core Network (CN) 90.

The conventional operation of networks 80, 90, and 100 is well-known in the art, and thus, not disclosed in detail herein. It is sufficient to understand that, in some embodiments of the present invention, the server(s) 84 and/or the server(s) 102 store information about landmarks and points of interest located near the user's current location that previous people found interesting. The cellular telephone 10 can leverage this information, along with information indicating the direction that user is currently looking (i.e., North, South, East, West, etc.), to predict a destination location for the user, and to provide information regarding landmarks along the way that the user may find interesting.

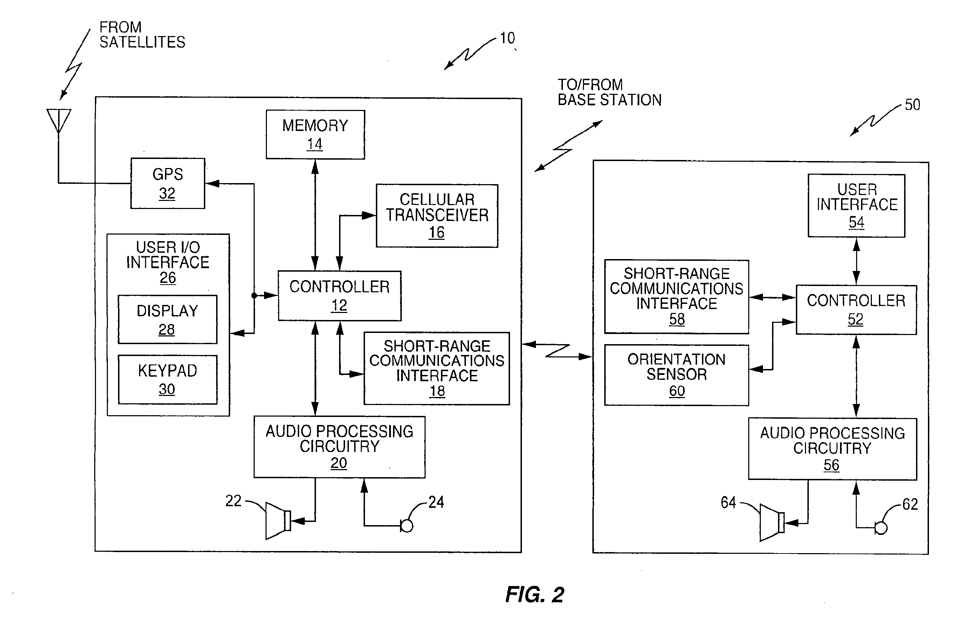

Figure 2 is a block diagram illustrating the component parts of a cellular telephone 10 and a headset 50 configured according to one embodiment of the present invention. Beginning with the cellular telephone 10, it comprises a controller 12, a memory 14, a cellular transceiver 16, a short-range communications interface 18, a audio processing circuitry 20, a user I/O interface 26, and a Global Positioning Satellite (GPS) receiver 32.

Controller 12 may be, for example, one or more general purpose or special purpose microprocessors that control the operation and functions of the cellular telephone 10 in accordance with program instructions and data stored in memory 14. In one embodiment of the present invention, the controller 12 executes a program to determine its current geographical coordinates, as well as a direction in which the user is looking. Based on this information, and on stored historical information regarding the places other users visited or found interesting, the controller 12 is programmed to identify one or more possible landmarks or points of interest that could be a destination location for the user. The controller 12 is also programmed to provide the user with information about these identified landmarks as well as with directions to a selected landmark.

Memory 14 represents the entire hierarchy of memory in cellular telephone 10, and may include both random access memory (RAM) and read-only memory (ROM). Memory 14 stores the program instructions and data required for controlling the operation and functionality of cellular telephone 10. In one embodiment of the present invention, memory 14 stores the instructions and data required by controller 12 for predicting a user's destination location and/or one or more landmarks along the way that the user might find interesting. In some embodiments, memory 14 stores all or most of the historical information needed to determine a user's destination location and/or the possible points of interest. In other embodiments, however, that information and data is stored in the server(s) 84, 102 and retrieved as needed by controller 12 and temporarily stored in memory 14.

Cellular transceiver 16 is a fully functional cellular radio transceiver for transmitting signals to and receiving signals from a base station or other access node in a wireless communications network. Those skilled in the art will appreciate that cellular transceiver 16 may implement any one of a variety of communication standards including, but not limited to, the standards known as the Global System for Mobile Communications (GSM), General Packet Radio Service (GPRS), Universal Mobile Telecommunication System (UMTS), TIA/EIA-136, cdmaOne (IS-95B), cdma2000, 3GPP Long Term Evolution (LTE), and Wideband CDMA (W-CDMA).

Cellular telephone 10 also includes a short-range communications interface 18, which in this embodiment, is a wireless interface that operates according to the BLUETOOTH protocol. As is known in the art, BLUETOOTH is a universal radio interface that enables two or more wireless devices to communicate wirelessly via short-range *ad hoc* networks. BLUETOOTH generally uses a polling based communication infrastructure to transmit and receive digital data between the wireless devices. In this embodiment, the communications interface 18 establishes a short-range communications link with a corresponding short-range communications interface 58 integrated within headset 50. Digital signals carrying voice and/or control signals are communicated between the cellular telephone 10 and headset 50 via the short-range communications link. Additionally, in one embodiment of the present invention, data and information regarding the orientation of the user's head is communicated from the headset 50 to the cellular telephone 10 via the established short-range link.

The audio processing circuit 20 processes audio signals for the cellular telephone 10 so that the user can communicate voice signals with a remote party. Particularly, the audio processing circuit 20 is communicatively connected to a speaker 22 and a microphone 24, which are generally part of the user I/O interface 26. Microphone 24 converts the user's speech into electrical audio signals for processing by audio processing circuit 20, which are then transmitted to the remote party via network 80. Speaker 22 converts the audio signals received from the remote party and provided by audio processing circuit 20 into audible sounds for rendering to the user.

The user interface 26 enables a user to exchange information with cellular telephone 10 and includes devices and controls that facilitate such interaction. Typically, the user interface 26 includes a display 28 (e.g., an LCD or touch-sensitive display) that allows the user to view information such as dialed digits, images, call status, menu options, and other service information. The user interface 26 will also include a keypad 30 that allows the user to enter digits and other alpha-numeric input.

The GPS receiver 32 is a receiver whose operation is well-known in the art. Generally, the GPS receiver receives navigational signals from a plurality of satellites 70 orbiting the Earth. From these signals, the GPS receiver can compute the geographical position of the cellular telephone 10, and thus, the user of cellular telephone 10, very accurately. For example, in some cases, the GPS receiver 32 can compute the current geographical position of the cellular telephone 10 to within a few meters. This position is then provided to the controller 12 for processing. In one embodiment, for example, the controller 12 uses the received coordinate information to determine where a user might be headed and/or to provide directions to the destination point.

The headset 50 comprises a controller 52, a user interface 54, an audio processing circuit 56 connected to a microphone 64 and a speaker 62, a short-range communications interface 58, and an orientation sensor 60. The user interface 54 typically comprises an on/off control button that powers-up (and down) the headset 50 responsive to the user actuating the control. The audio processing circuitry 56 receives electrical audio signals from the microphone 64 and converts them to digital signals for the short-range communications interface 58 to transmit to the short-range communications interface 18. The audio processing circuitry 56 also receives digital audio signals from the cellular telephone 10 via short-range communications interface 58, and processes those signals for rendering to the user on speaker 62. Typically, the speaker 62 and the microphone 64 are positioned at opposite ends of a boom that fits to the user's head. The short-range communications interface 58 comprises, for example, a corresponding BLUETOOTH transceiver that communicates wireless signals with the BLUETOOTH transceiver of cellular telephone 10. In one embodiment of the present invention, the short-range communications interface 58 communicates orientation information measured by the orientation sensor 60, and sends it to cellular telephone 10.

The orientation sensor 60 may comprise any sensor known in the art able to detect the movement of a user's head while he or she is wearing the device, and provide an indication of the direction in which the user is currently looking or facing. Some examples of orientation sensors 60 that are suitable for use in headset 50 include, but are not limited to, gyroscopes, accelerometers, and compasses. The orientation sensor 60 may provide the orientation information to the controller 12 periodically, or may only provide the information to controller 12 responsive to receiving a valid message requesting the orientation information.

In one embodiment, the orientation sensor 60 comprises a solid-state compass. As is known in the art, a solid state compass is a navigational device used to determine direction relative to the surface of the Earth. Generally, solid-state compasses are constructed from a variety of highly sensitive magnetic field sensors that output digital or analog signals proportional to their orientation relative to the Earth's surface. These signals are sent to the controller 52, which upon receipt, employs well-known trigonometric techniques on the signals to compute a corresponding heading (i.e., the direction in which the user is looking). The controller 52 then periodically sends the corresponding heading information to the controller 12 via the short-range link. Alternatively, the controller 52 could transmit the heading information to controller 12 in a message responsive to receiving a request for the information from controller 12.

As previously stated, the present invention determines the geographical position for the cellular telephone 10 as well as a direction or heading in which the user is currently looking. Based on this information, the controller 12 in the cellular telephone 10 identifies one or more locations that are nearby the user's current position and predicts one of those points as being a destination location for the user. In some embodiments, the present invention also provides information regarding these one or more locations for the user as they could be of interest to the user.

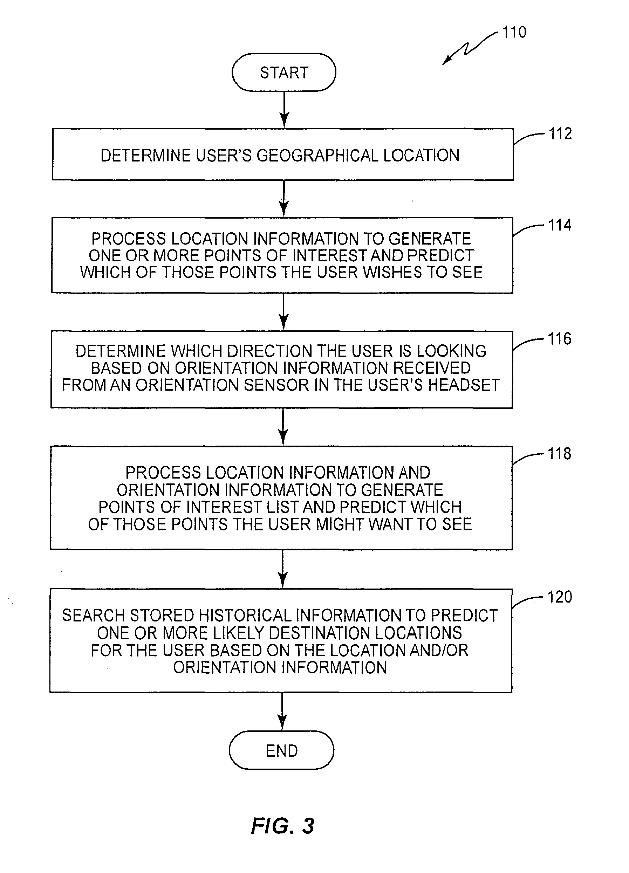

Figure 3 is a flow chart illustrating a method 110 performed by cellular telephone 10 according to one embodiment of the present invention. Method 110 begins with cellular telephone 10 determining its geographical location (box 112). This may be accomplished, for example, using the GPS receiver 32 as is known in the art. Alternatively, for those cellular telephones that are not equipped with a GPS receiver, server 84 or BS 82 in network 80 can compute the geographical position of the cellular telephone using well-known triangulation techniques, and the provide the position to the cellular telephone 10. Once the cellular telephone 10 has determined its geographical position, controller 12 generates a list of one or more points of interest for the user, and predicts which of those points or locations the user might wish to see or visit (box 114).

Generating the points of interest and predicting which point a user might wish to see can be accomplished using a variety of different methods. In one embodiment, however, the present invention leverages the fact that people generally pass or visit the same locations when enroute to a destination location. Therefore, the cellular telephones of many people can, over time, collect data indicating what locations their users visited or passed while previously at or near the user's current position and store it in a common database. This "historical data" is collected and stored in a database by the network (e.g., network 80, 100). The database may be located in a single network server (e.g., server 84). However, in one preferred embodiment, the database is distributed over many servers (e.g., servers 102) in cloud network 100. Then, as the user travels, controller 12 compares the user's current geographic position to the stored positions of the previous people. Based on this comparison, controller 12 generates a list of possible locations that are near the user and that the user may be interested in seeing or visiting.

Although helpful, the use of a user's current geographical position, by itself, may not always be enough information to generate an accurate list of locations or accurately predict to which of those the user might go. Information about what a user is looking at would be particularly helpful in determining these locations. Therefore, in one embodiment, the present invention also utilizes the orientation information provided by the orientation sensor 60 disposed in headset 50. Using this information, in addition to the user's current geographical position 60, allows the present invention to more accurately predict where the user could be headed and/or what locations the user may find interesting along the way. Therefore, when controller 12 receives a signal from the orientation sensor 60 indicating which direction the user is looking (box 116), controller 12 processes that orientation information along with the location information to determine the contents of the list for the user and predict which of those points the user wishes to see (box 118). For example, if controller 12 determines that the user is looking generally in a westerly direction, the controller 12 need only to generate a list that includes those locations that are proximate the user's current location in that westerly direction.

In addition generating the points of interest list, the present invention also configures controller 12 to predict a likely destination location for the user (i.e., which of the points of interest the use wishes to see). In one embodiment, controller 12 will compare both the geographical position of the user and the orientation information defining the direction of the user's head to the saved historical data stored in the database. The controller 12 then uses the results of the comparison to generate the list and predict which of the points of interest the user is most likely headed towards (box 120).

For example, consider a situation where a landmark or point of interest is located to the west of the user. If the user looks at the landmark, controller 12 would receive signals from the orientation sensor 12 indicating that the user was facing or looking towards the west. In this case, the controller 12 would only have to obtain information from the database for the locations or points of interest that lie to the west of the user. Then, if the user changed directions and began moving east or north, for example, the controller 12 would update the points of interest based on the updated orientation signals received from the orientation sensor 60.

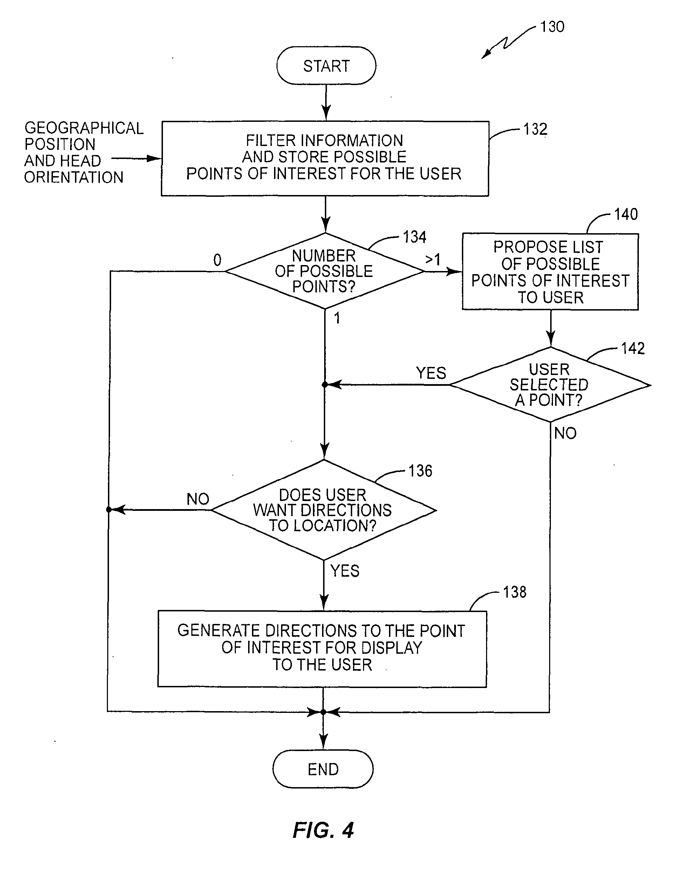

Figure 4 is a flow diagram illustrating a method 130 of generating the list of points of interest according to one embodiment of the present invention. In the embodiment explained below, the method 130 is performed at controller 12. However, this is for illustrative purposes only. Because there could be a vast amount of stored data collected from many different sources, method 130 may be performed at one or more of the servers 84 or 102 in either of the corresponding networks 80, 100. In these cases, the cellular telephone 10 could provide the information to the servers, and then receive the list in response.

Method 130 begins when controller 12 obtains the historical data from the database, and utilizes the required information to generate and store a list containing one or more points of interest for the user (box 132). As previously stated, the requisite information needed by controller 12 includes the geographical position of the user and the orientation of the user's head (i.e., the direction in which the user is looking). Particularly, the controller 12 will compare this information against the recorded positions and orientations of previous users to generate a list of points or locations that the current user may find interesting.

Based on the number of possible points that are added to the list, controller 12 will perform a different function. Particularly, if no locations are found or added to the list, the process ends. If only one (1) location is found, however, the controller 12 will generate and display a user message to determine whether the user wants directions to the location (box 136). If the user does not want directions, the process ends. If the user does want directions, however, controller 12 will generate and display the directions to the point of interest from the current location of the user (box 138). Alternatively, where controller 12 is not configured to generate the directions, controller 12 could generate a request for the directions from a third-party server accessible to the cellular telephone 10.

If there are multiple possible points of interest on the list (box 134), the controller 12 could generate and display the list to the user and propose to the user that he or she visit a particular location (box 140). The recommendation to visit a particular location could be based, for example, on the historical data and the user's current position and orientation. Additionally, or alternatively, the recommendation to visit a particular location may be based on the availability of coupons, discounts, or other commercial proposals received by businesses that are on the way to a destination. Other methods of placing possible points of interest on the list and/or recommending a particular location are also possible. If desired, the user can then select a particular point of interest from the list using keypad 30, for example (box 142). For a selected location, controller 12 will ask the user if he or she would like directions to that selected location (box 136) and generate or otherwise obtain those directions as previously stated.

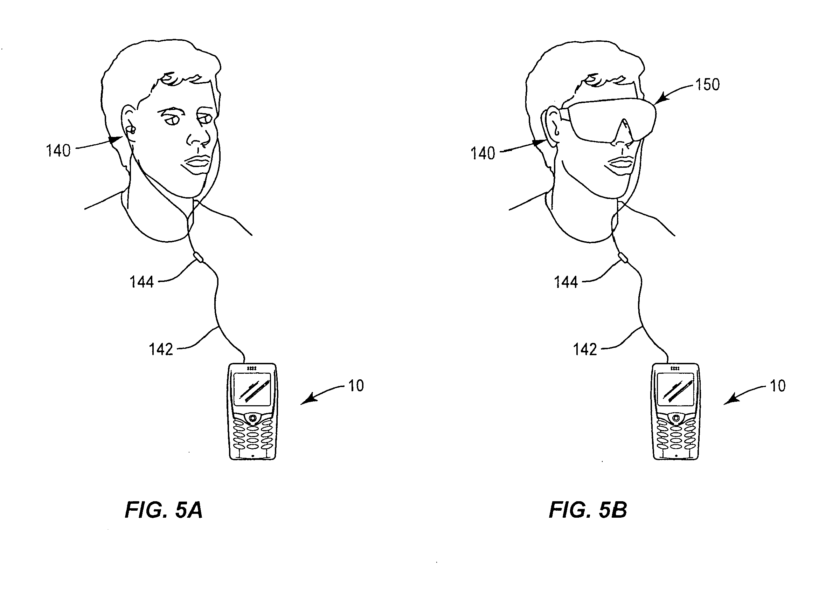

The present invention may, of course, be carried out in other ways than those specifically set forth herein without departing from essential characteristics of the invention. For example, the hands-free headset used with the present invention need not be a wireless headset 50 that communicates over a wireless communications link with cellular telephone 10. In other embodiments, such as those seen in Figures 5A and 5B, the hands-free headset comprises a headset 140 that connects to the cellular telephone 10 (or other suitable device) via a cable 142. In these cases, the short-range communication interfaces 18, 58 may comprise, for example, corresponding Universal Serial Bus (USB) interfaces. An external microphone 144 may be disposed along the cable 142 as is known in the art to capture the user's voice in a hands-free mode of operation.

In such embodiments, the orientation sensor 60 and other components may still be integrated into the headset 140, such as in the ear bud, for example, to facilitate determining a direction in which the user is looking. The cellular telephone 10 would then receive this information and, based on the information, would generate and display directions to various points of interest as previously described.

In addition, the present invention is not limited to simply outputting directions and other navigational information to the display 28 of cellular telephone 10. In at least one embodiment, seen in Figure 5B, the user has a head-mounted display (HMD) 150 that incorporates the headset 140. As is known in the art, HMDs are display devices that are worn by a user. Figure 5B illustrates the HMD 150 as being a pair of glasses; however, in other embodiments, the HMD 150 may comprise a helmet. In operation, the HMD 150 connects to the cellular telephone 10 using a cable 142. Cable 142 may be, for example, a Universal Serial Bus (USB) cable that can communicate both data and audio to and from the cellular telephone 10 via corresponding short-range communication interfaces 18, 58. With this capability, HMD 150 may receive text and/or image information from the cellular telephone 10 and display that information on one or more small displays embedded in the lenses 152. The displays may be miniaturized LCD displays, for example, and superimpose the received text and/or images on the lenses such that the information does not block the "real world" view. Thus, menu choices, directions to a recommended location, or information about a particular location, for example, may be displayed by the HMD 150 for the user while still allowing the user to view the location and the rest of the world around him or her.

In addition, the present invention may be equipped for voice-to-text and text-to-voice conversion. This would enable embodiments, for example, in which the cellular device 10 could convert received voice or other audio into text for display on HMD 140 (or display 28). Additionally, it could allow the cellular device 10 to convert the user's voice into text and use those text phrases as commands for operating the cellular device 10. Therefore, the present embodiments are to be considered in all respects as illustrative and not restrictive, and all changes coming within the meaning and equivalency range of the appended claims are intended to be embraced therein.

## Claims

1. A wireless communication device configured to communicate with a headset worn by a user, the wireless communication device comprising:
a short-range communication interface configured to receive orientation information from the headset via a short-range communication link; and
a controller configured to:
determine a current geographical position of the user;
determine which direction the user is looking based on the orientation information; and
predict a destination location for the user based on the user's current geographical position and the determined direction information.

2. The wireless communication device of claim 1 wherein the controller is configured to receive the orientation information via the short-range communication interface from an orientation sensor integrated within the headset.

3. The wireless communication device of claim 1 wherein the controller is configured to:
send a request for the orientation information to the headset via the short-range communication interface; and
receive the orientation information from the orientation sensor via the short-range communication interface responsive to the request..

4. The wireless communication device of claim 1 wherein the controller is further configured to:
generate a first list of locations comprising one or more points of interest based on the user's current geographical position;
filter the generated list of locations based on the orientation information to identify a second list of locations comprising one or more points of interest; and
select the destination location for the user from the second list of locations based on the user's current geographical position and the orientation information.

5. The wireless communication device of claim 1 further comprising a display, and wherein the controller is further configured to generate and display directions to a selected destination location responsive to user input.

6. The wireless communication device of claim 1 wherein the controller is further configured to generate directions to a selected destination location and send the directions to a display associated with a head-mounted display.

7. A method of predicting a destination location for a user comprising:
establishing a short-range communication link with a headset worn by a user;
determining which direction the user is looking based on orientation information received from the headset via the established short-range communication link; and
predicting a destination location for the user based on a current geographical position of the user and on the orientation information received from the headset.

8. The method of claim 7 further comprising:
generating a request to obtain the orientation information from an orientation sensor integrated within the headset;
sending the request to the orientation sensor; and
receiving the orientation information from the orientation sensor responsive to the request.

9. The method of claim 7 wherein predicting a destination location for the user comprises:
generating a first list of locations comprising one or more points of interest based on the user's current geographical position for the user;
filtering the first list of locations based on the orientation information received from the headset to identify a second list of locations for the user; and
selecting a destination location from the second list of one or more points of interest based on the user's current geographical position and the orientation information.

10. The method of claim 7 further comprising generating and displaying directions to a selected destination location responsive to user input.

11. A hands-free headset for a wireless communication device, the headset comprising:
a microphone;
a speaker;
an integrated direction sensor configured to generate orientation information indicating which direction a user is looking based on a detected orientation of the user's head; and
a short-range communication interface configured to communicate the orientation information to a wireless communication device.

12. The hands-free headset of claim 11 further comprising a controller configured to cause the short-range communication interface to transmit the orientation information to the wireless communication device responsive to receiving a request for the orientation information from the wireless communication device.

13. The hands-free headset of claim 11 further comprising a head-mounted display to display text and/or images received from the wireless communication device.

14. A method of predicting a destination location for a user of a wireless communication device, the method comprising:
establishing a short-range communication link between a user's wireless communication device and a hands-free headset worn by the user;
detecting an orientation of the user's head using a sensor in the headset;
generating orientation information indicating which direction the user is looking based on the detected orientation of the user's head; and
transmitting the orientation information to the wireless communication device via the short-range communication link.

15. The method of claim 14 wherein transmitting the orientation information comprises transmitting the orientation information to the wireless communication device responsive to receiving a request for the orientation information from the wireless communication device.
